# EUROPÄISCHE PATENTSCHRIFT

(11) **EP 2 161 337 B1**
(45) Veröffentlichungstag und Bekanntmachung des Hinweises auf die Patenterteilung: **21.09.2011**
(21) Anmeldenummer: 09009305.5
(22) Anmeldetag: 17.07.2009
(51) Int. Cl.: C12N 15/10, C12Q 1/68

(54) **Verfahren zur Isolierung von Nukleinsäuren aus nukleinsäurehaltigen Proben sowie ein dazu geeignetes Testkit**
Process for isolating nucleic acids from a nucleic acid-containing sample and a testkit
Méthode pour l'isolement d'acides nucléiques à partir d'un echantillon contenant des acides nucléiques et un kit de test

(30) Priorität: 28.08.2008 DE 102008044721
(43) Veröffentlichungstag der Anmeldung: 10.03.2010
(73) Patentinhaber: Forschungszentrum Jülich GmbH, 52425 Jülich (DE)
(72) Erfinder: Martin, Sven, 40822 Mettmann (DE)

(56) Entgegenhaltungen:
- US-A- 5 234 809
- ANONYMOUS: "RNeasy Mini Handbook - English (PDF)" [Online] 1. Mai 1999 (1999-05-01), QIAGEN , XP002544114 Gefunden im Internet: URL:http://www1.qiagen.com/Products/RnaSta bilizationPurification/RNeasySystem/RNeasy PlantMini.aspx?rp=1000292&rpg=0#Tabs=t2> [gefunden am 2009-08-31] * das ganze Dokument * * Seite 5 *
- BOOM R ET AL: "RAPID AND SIMPLE METHOD FOR PURIFICATION OF NUCLEIC ACIDS" JOURNAL OF CLINICAL MICROBIOLOGY, WASHINGTON, DC, US, Bd. 28, Nr. 3, 1. März 1990 (1990-03-01), Seiten 495-503, XP000607196 ISSN: 0095-1137
- "MODIFICATION OF THE TRI REAGENT PROCEDURE FOR ISOLATION OF RNA FROM POLYSACCARIDE- AND PROTEOGLYCAN-RICH SOURCES" BIOTECHNIQUES, INFORMA LIFE SCIENCES PUBLISHING, WESTBOROUGH, MA, US, Bd. 19, Nr. 6, 1. Dezember 1995 (1995-12-01), Seiten 942-945, XP008050027 ISSN: 0736-6205
- ANONYMOUS: "High-Salt Solution for Precipitation (Plant)" [Online] 1. Mai 2008 (2008-05-01), TAKARA BIO INC. , XP002544115 Gefunden im Internet: URL:http://catalog.takara-bio.co.jp/en/PDF Files/9193_DS_e.pdf> [gefunden am 2009-08-31] * das ganze Dokument *

## Beschreibung

Die Erfindung betrifft ein Verfahren zur Isolierung von Nukleinsäuren aus nukleinsäurehaltigen Proben, die einen hohen Gehalt an Primär- und Sekundärmetaboliten oder Polysacchariden aufweisen, sowie ein dazu geeignetes Testkit.

Die Isolierung von Nukleinsäuren spielt in der gesamten Biologie eine bedeutende Rolle. Es existieren zahlreiche Verfahren, welche die Isolierung von Nukleinsäuren ermöglichen. Das Patent US 5,234,809, Boom R. et al ("Rapid and simple method for purification of nucleic acids" Journal of clinical Microbiology, Mar. 1990; 495-503) sowie das "RNeasy Mini-Handbook- Englisch (PDF)" von Qiagen (online Mai 1999:
http://www.qiagen.com/produets/mastabilizationpurification/rneasysystem/measymini.aspx# Tabs=t2, Anonymus/Qiagen) beschreiben einen Prozess zur Isolierung von Nukleinsäuren aus nukleinsäurehaltigem Ausgangsmaterial durch Inkubation des Ausgangsmaterials mit einem chaotropen Puffer und einer DNA-bindenden festen Phase. Die DNA-bindende feste Phase besteht dabei aus silikatischen mineralischen Partikeln. Die chaotropen Puffer realisieren sowohl die Lyse des Ausgangsmaterials als auch die Bindung der Nukleinsäuren an die feste Phase. Weitere Verfahren beschreiben die chromatographische Reinigung und Trennung von Nukleinsäuregemischen aus einer Lösung, die eine hohe Salzkonzentration und eine hohe Alkoholkonzentration aufweist. Die Lösung wird wiederum mit einem Trägermaterial zur Anbindung der Nukleinsäuren in Kontakt gebracht, nachfolgend wird das Trägermaterial mit an sich bekannten alkoholischen Waschpuffern gewaschen und die gebundene Nukleinsäure mittels Wasser oder einem Niedrigsalzpuffer wieder vom Trägermaterial abgelöst.

Es ist weiterhin ein Verfahren zur Isolierung von Nukleinsäuren bekannt, bei dem eine Mischung aus Phenol, Natrium/Ammonium-Thiocyanat und Guanidinthiocyanat verwendet wird (TRI Reagenz). Bei diesem Verfahren wird die Probe in dem TRI Reagenz homogenisiert und anschließend organisches Lösungsmittel zugegeben, so dass sich eine wässrige Phase enthaltend RNA, eine organische Phase enthaltend Proteine sowie eine Interphase enthaltend DNA bildet. Die gewünschte RNA kann dann durch Zugabe von Alkohol und anschließende Sedimentation der RNA erhalten werden. Bei der Aufreinigung von Nukleinsäuren aus Proben, die reich an Polysacchariden und Proteoglycane sind, wird zur Verbesserung der RNA Isolierung nach Abtrennung der wässrigen RNA-haltigen Phase eine Hochsalzlösung zugegeben, bestehend aus 1,2 M NaCL und 0,8 M Natriumcitrat (P. Chomczynski; K. Mackey: "Modification ofthe TRI reagent ™ procedure for isolation of RNA from polysaccharide- and proteoglucan-rich sources", BioTechniques, 19, 942-945,1995; Anonymous: "High-salt solution for precipitation (plant)", online: http.//catalog.takara-bio.co.jp/en/PDF, Takara Bio Inc., 2008).
Weitere Verdahren beschreiben die chromatographische Reinigung und Trennung von Nukleinsäuregemischen aus einer Lösung, die eine hohe Salzkonzentration und eine hohe Alkoholkonzentration aufweist. Die Lösung wird wiederum mit einem Trägermaterial zur Anbindung der Nukleinsäuren in Kontakt gebracht, nachfolgend wird das Trägermaterial mit an sich bekannten alkoholischen Waschpuffern gewaschen und die gebundene Nukleinsäure final mittels Wasser oder einem Niedrigsalzpuffer wieder vom Trägermaterial abgelöst.

Mit den bisher bekannten Verfahren ist eine Isolation von "Gesamt-Nukleinsäuren", d.h. alle Typen von RNA und DNA, aus Proben, die einen hohen Gehalt an Primär-bzw. Sekundärmetaboliten aufweisen, zeitlich sehr arbeitsaufwendig bis nahezu unmöglich. Dies betrifft sowohl die Reinheit als auch die Menge an isolierter Nukleinsäure.

Es ist daher Aufgabe der Erfindung ein Verfahren zu schaffen, mit dem eine einfache und schnelle Isolierung von Nukleinsäure aus nukleinsäurehaltigen Proben mit, gegenüber dem Stand der Technik, höheren Ausbeuten möglich wird. Es ist weiterhin Aufgabe der Erfindung, ein Testkit bereit zu stellen, mit dem dies erreicht werden kann.

Unter nukleinsäurehaltigen Proben sollen Proben von tierischen Zellen, Bakterien, Hefen, Pilzen und insbesondere pflanzlichen Zellen, wie beispielweise Zellen aus *Nicotiana tabacum, Populus spec., Hordeum vulgare, Triticum spec., Zea mays* und *Oryza spec*., verstanden werden und die auf Grund von äußeren Einflüssen, wie biespielweise künstlichen genetischen Veränderungen oder Einfluss von Stress, z.B. in Form von Aussetzen der Pflanzen mit einer CO₂ Konzentration von 800 ppm oder einem Stickstoffgehalt von 0,5 mM NO₃⁻, einen gegenüber unbeeinflussten tierischen Zellen, Bakterien, Hefen, Pilzen und pflanzlichen Zellen, höheren Anteil an Primär- und Sekundärmetaboliten aufweisen oder bei denen eine Nukleinsäureisolierung auf Grund eines hohen Anteils an Polysacchariden auch ohne Stresseinfluss erschwert ist (wie beispielweise eine RNA Isolierung aus *Populus spec.*). So kann das Verfahren und das Testkit beispielweise auch zur Isolierung von Nukleinsäuren aus Pharmapflanzen oder gentechnisch veränderten pflanzlichen Zellen oder tierischen Zellen geeignet sein, die auf Grund der genetischen Veränderung einen höheren Anteil an Kohlehydraten produzieren gegenüber den natürlich vorkommenden Zellen.

Ausgehend von Oberbegriff des Anspruchs 1 wird die Aufgabe erfindungsgemäß gelöst mit den im kennzeichnenden Teil des Anspruchs 1 angegebenen Merkmalen. Weiterhin wird die Aufgabe, ausgehend vom Oberbegriff des Anspruchs 7, erfindungsgemäß gelöst mit den im kennzeichnenden Teil der Anspruchs 7 angegebenen Merkmalen.

Es hat sich gezeigt, dass es mit dem erfindungsgemäßen Verfahren möglich wird, insbesondere aus pflanzlichen Zellen eine gegenüber dem Stand der Technik höhere Ausbeute an Nukleinsäuren zu isolieren. Als besonders geeignet hat sich das Verfahren zur Isolation von Gesamt RNA erwiesen.

Mit dem erfindungsgemäßen Verfahren wird es beispielsweise auch möglich, eine vereinfachte Isolierung der RNA mit einem geringeren Verbrauch an toxischen Chemikalien wie z. B. Chloroform, Phenol, Bromchlorpropan, zu erreichen.

Die vorliegende Erfindung betrifft ein Verfahren zur Isolierung von Nukleinsäuren aus nukleinsäurehaltigen Proben, die einen hohen Gehalt an Primär- und Sekundärmetaboliten oder Polysacchariden aufweisen, umfassend einen mechanischen Aufschluss der Proben mit bekannten Verfahren, Zugabe von einem chaotropen Denaturierungsmittel zur Probe, Homogenisieren der lysierten Probe und Entfernen der Zellreste durch Zentrifugation, Zugabe von Alkohol, Abtrennung der Nukleinsäuren durch Bindung der Nukleinsäuren an einen mineralischen Träger, Waschen sowie abschließendes Waschen und Eluieren der adsorbierten Gesamtnukleinsäure vom mineralischen Träger nach bekannten Verfahren, das dadurch gekennzeichnet ist, dass vor dem Eluieren der adsorbierten Gesamtnukleinsäure eine Zugabe einer 0,8M NaCitrat/1,2M NaCl-Lösung erfolgt.

Um die Proben mechanisch aufzuschließen, können alle für die jeweiligen Proben geeigneten, nach dem Stand der Technik bekannten Verfahren eingesetzt werden. Um pflanzliche Zellen mechanisch aufzuschließen, können die tiefgekühlten Zellen beispielsweise unter flüssigem Stickstoff fein gemörsert werden. Eine weitere Möglichkeit ist die Verwendung eines Mikropistills in einem Reaktionsgefäß, wie z. B. einem Eppendorfgefäß.

Als chaotropes Denaturierungsmittel kann beispielsweise Guanidinhydrochlorid, Guanidinium-Isothiocyanat oder Guanidinium-Thiocyanat eingesetzt werden. Dies kann beispielsweise durch Zugabe von RLC-Puffer der Firma Qiagen oder LG-Puffer der Firma Roboklon erfolgen. Durch Zugabe des Denaturierungsmittels wird die Probe lysiert.

Nach Zugabe des Denaturierungsmittels wird die Probe homogenisiert und gleichzeitig Zellreste/-trümmer entfernt. Dies kann beispielsweise durch Zentrifugieren in einem Plastik-Reaktionsgefäß (z. B. Eppendorfgefäß) oder mit Hilfe einer Trennsäule, die ebenfalls zentrifugiert wird, erreicht werden.

Die Zugabe von Alkohol bewirkt, dass die Bedingungen in der Reaktionslösung so eingestellt werden, dass die Nukleinsäuren anschließend gut an den mineralischen Träger bzw. die Trennsäulen binden. Als besonders geeignet hat sich die Zugabe von Ethanol erwiesen.

Zur Abtrennung der Nukleinsäuren werden diese mittels eines mineralischen Trägers spezifisch gebunden. Der mineralische Träger kann beispielsweise aus einem silikatischen Material bestehen. Zur Abtrennung der Nukleinsäuren können alle nach dem Stand der Technik bekannten Trennsäulen eingesetzt und je nach Bedarf des abzutrennenden Nukleinsäuretyps (DNA, RNA, mRNA, einzelsträngig, doppelsträngig usw.) ausgewählt werden.

Zur weiteren Reinigung können anschließend Waschschritte durchgeführt werden. Dies kann beispielsweise durch einen geeigneten nach dem Stand der Technik bekannten Waschpuffer (wie z. B. einen RW1-Puffer der Firma Qiagen, Wash-RB 1-Puffer der Firma Roboklon) erfolgen, der alle oder einen großen Teil der Verunreinigungen von der Trennsäule wäscht, so dass nur die Nukleinsäuren gebunden bleiben. Es können je nach Probentyp und gewünschter Reinheit der Nukleinsäure ein bis beispielsweise 3 Waschschritte durchgeführt werden.

Anschließend wird eine Hochsalzlösung bestehend aus Natriumcitrat und NaCl zugegeben, um Verunreinigungen durch Primär- und Sekundärmetabolite zu entfernen. Zu den Primär und Sekundärmetaboliten zählen beispielsweise Poly- und Oligosaccharide sowie Polyphenole. Als Hochsalzlösung eignet sich besonders eine Zusammensetzung aus 0,8 M/1,2M NaCitrat/NaCl-Lösung (= HiSS®- Lösung).

In einem weiteren Schritt werden die Nukleinsäuren durch geeignete Waschpuffer (z. B. RW1-Puffer, RPE-Puffer der Firma Qiagen; Wash-RBW der Firma Roboklon) von der Hochsalzlösung und restlichem Ethanol gereinigt. Mit Hilfe von Waschlösungen (z. B. nukleinsäurefreies Wasser) können die Nukleinsäuren anschließend von der Säule eluiert werden.

Die Erfindung betrifft weiterhin ein Testkit, welches für das oben beschriebene Verfahren geeignet ist.

Dieses Testkit umfasst
a) Puffer, enthaltend Guanidinhydrochlorid, Guanidinisothiocyanat oder Guanidinthiocyanat
b) Ethanol
c) Trennsäule für Nukleinsäuren
d) Waschpuffer
e) NaCitrat/NaCL-Lösung (= HiSS, Hochsalzlösung)
f) Auffanggefäße

Vorteilhafte Weiterbildungen sind in den Unteransprüchen angegeben.

Im Folgenden soll ein Ausführungsbeispiel angegeben werden, welches das erfindungsgemäβe Verfahren und das erfindungsgemäße Testkit näher erläutert.

### Isolierung von Gesamt-RNA aus Pflanzenproben mit Hilfe des Rneasy^{®} Plant Mini Kits (Oiagen) mit Hochsalzlösung (HiSS)

Da β-Mercaptoethanol giftig ist, sollte die gesamte Isolation unter einem Abzug stattfinden. Die Proben sollten, um die Gefahr des Verdaus durch RNasen zu minimieren, nach der Elution auf Eis gekühlt werden.

### Benötigtes Material und Geräte:

- Mörser und Pistill
- flüssiger Stickstoff
- 1,5 ml und 2 ml Reaktionsgefäße (z. B. Eppendorf Caps)
- 96-100% iger Ethanol (EtOH)
- 14,3 M β-Mercaptoethanol
- sterile, RNase-freie Pipettenspitzen
- Tischzentrifuge (nicht kälter als 20 °C)
- Einmalhandschuhe
- alle Schritte sollen bei Raumtemperatur durchgeführt werden (20 -25° C)

1. Abwiegen des Pflanzenmaterials. Es sollten nicht mehr als 100 mg sein.
   Das Auftauen der Probe sollte vermieden werden. Zügig mit Schritt 2 fortfahren.
2. Abgewogenes Material in Mörser überführen und unter flüssigem Stickstoff äußerst fein mörsern. Das Pulver in ein, mit flüssigem Stickstoff vorgekühltes, 1,5 ml Reaktionsgefäß überführen.
   Das Auftauen der Probe sollte vermieden werden. Zügig mit Schritt 3 fortfahren.
3. 450 µl "RLC"-Puffer hinzufügen und gut vortexen.
   Solange vortexen, bis eine homogene Lösung zu erkennen ist.
4. Das Lysat auf die QIAshredder-Säule (lila), befindlich in einem 2 ml Reaktionsgefäß, pipettieren und für 2 min bei maximaler Geschwindigkeit zentrifugieren. Der Überstand wird vorsichtig, ohne das Pellet zu zerstören, in ein 1,5 ml Reaktionsgefäß überführt.
5. Das 0,5fache des Lysatvolumens, meist zwischen 220-230 µl, EtOH (96-100 %) dazugeben. Sofort durch auf- und abziehen mit der Pipette mischen.
   Zügig mit Schritt 6 fortfahren.
6. Die gesamte Lösung (meist 650 µl), inklusive des sich eventuell gebildeten Präzipitats, wird auf die RNeasy-Säule (rosa), befindlich in einem 2 ml Reaktionsgefäß, gegeben. Zentrifugation für 15 Sekunden bei ≥ 8000 x g (≥ 10000 rpm). 20 Sekunden bei 10000 rpm sind auch möglich
   Der Durchfluss wird verworfen. Das 2 ml Reaktionsgefäß wird in Schritt 7 weiter verwendet.
7.
   7.1 Es wird eine 0,8 M NaCitrat / 1,2 M NaCl-Lösung (HiSS^{®}) hergestellt.
   7.2 Es werden 350 µl "RW1"-Puffer auf die RNeasy-Säule pipettiert. Zentrifugation für 15 Sekunden bei ≥ 8000 x g (≥ 10000 rpm). *20 Sekunden bei 10000 rpm sind auch möglich*.
      Der Durchfluss wird verworfen. Das 2 ml Reaktionsgefäß wird weiter verwendet.
   7.3 Es werden 250 µl NaCitrat/NaCl-Lösung auf die RNeasy-Säule pipettiert.
      Inkubation bei Raumtemperatur (20-30°C) für 15 Minuten.
   7.4 Es werden erneut 250 µl NaCitrat/NaCl-Lösung auf die RNeasy-Säule pipettiert.
      Zentrifugation für 15 Sekunden bei ≥ 8000 x g (≥ 10000 rpm).
      20 Sekunden bei 10000 rpm sind auch möglich.
      Der Durchfluss wird verworfen. Das 2 ml Reaktionsgefäß wird weiter verwendet.
   7.5 Es werden erneut 350 µl "RW1"-Puffer auf die RNeasy-Säule pipettiert.
      Zentrifugation für 15 Sekunden bei ≥ 8000 x g (≥ 10000 rpm).
      20 Sekunden bei 10000 rpm sind auch möglich.
      Der Durchfluss und das 2 ml Reaktionsgefäß werden verworfen.
8. Die RNeasy-Säule wird in ein neues 2 ml Reaktionsgefäß (im Kit enthalten) überführt.
   Es werden 500 µl "RPE"-Puffer auf die Säule pipettiert.
   Zentrifugation für 15 Sekunden bei ≥ 8000 x g (≥ 10000 rpm).
   20 Sekunden bei 10000 rpm sind auch möglich.
   Der Durchfluss wird verworfen. Das 2 ml Reaktionsgefäß wird weiter verwendet.
9. Es werden erneut 500 µl "RPE"-Puffer auf die Säule pipettiert.
   Zentrifugation für 2 Minuten bei ≥ 8000 x g (≥ 10000 rpm).
   Die Silica-Säule wird dadurch getrocknet.
   Der Durchfluss und das 2 ml Reaktionsgefäß werden verworfen.
10. Die RNeasy-Säule wird in einem neuen 2 ml Reaktionsgefäß platziert.
   Zentrifugation für 1 Minute bei Höchstgeschwindigkeit der Zentrifuge.
   Eventuell noch vorhandenes EtOH wird abzentrifugiert.
11. Zur Elution der RNA wird die RNeasy-Säule in ein neues 1,5 ml Reaktionsgefäß platziert.
   Es werden 30-50 µl RNase-freies Wasser auf die Säule gegeben.
   Ca. 1 Minute abwarten.
   Zentrifugation für 1 Minuten bei ≥ 8000 x g (≥ 10000 rpm).
   Als besonders geeignet hat sich eine Elution mit 2x35µl RNase-freiem Wasser erwiesen. Das Endvolumen beträgt dann ca. 60 µl.

Im Folgenden werden einige Versuchsergebnisse zur RNA Isolation aufgeführt, die mit dem erfindungsgemäßen Verfahren im Vergleich mit einem nach dem Stand der Technik bekannten Verfahren durchgeführt wurden.

Beispielhaft wurde die RNA aus *Nicotiana spec.* und *Populus spec.* isoliert und sowohl die Reinheit als auch die Nukleinsäurekonzentration der RNA im Vergleich mit der erfindungsgemäßen Methode und einer nach dem Stand der Technik bekannten Methode (z. B. RNeasy Kit von Qiagen) bestimmt.

Die Bestimmung der Nukleinsäurekonzentration erfolgte spektrophotometrisch. Dabei wurden die Extinktionen der RNA-haltigen Lösungen bei 230 nm, 260 nm und 280 nm bestimmt. Die Konzentration der RNA in der Lösung wurde über die Extinktion der RNA-haltigen Lösung bei 260 nm ermittelt. Die Quotienten 260/280 nm sowie 260/230 nm geben Auskunft über den Reinheitsgrad der RNA. Ersterer sollte ≥ 1,7 und letzterer sollte ≥ 2,0 sein.

Die folgenden Tabellen zeigen die Ergebnisse der untersuchten Proben:

**Tabelle 1:**

| RNA Isolation aus vier unterschiedlichen Proben von *Nicotiana spec.* Jeweils mit dem erfindungsgemäßen Verfahren (mit HiSS= Hochsalzlösung wurde zugegeben) und einem nach dem Stand der Technik bekannten Verfahren (ohne HiSS = ohne Hochsalzlösung, mit dem RNeasy Mini Kit zur RNA Isolation von Qiagen) durchgeführt | | | | | | |
|---|---|---|---|---|---|---|
| **Probe** | 1 | 2 | 3 | 4 | 5 | 6 |
| | **OD₂₆₀/OD₂₃₀** | **OD₂₆₀/OD₂₈₀** | **c [µg/µl]** | **c (ng/mg FW)** | **Ausbeute-erhöhung (%)** | **Faktor** |
| **1)** ohne HiSS | 2,59 | 1,58 | 0,81 | 9,21 | | |
| mit HiSS | 2,60 | 1,61 | 1,24 | 17,41 | 88,96 | 1,89 |
| **2)** ohne HiSS | 2,50 | 1,61 | 0,41 | 4,32 | | |
| mit HiSS | 2,60 | 1,62 | 0,50 | 6,15 | 42,26 | 1,42 |
| **3)** ohne HiSS | 2,56 | 1,79 | 0,09 | 0.90 | | |
| mit HiSS | 2,78 | 1,74 | 0,13 | 1,21 | 34,84 | 1,35 |
| **4)** ohne HiSS | 3,08 | 1,74 | 0,21 | 2,16 | | |
| mit HiSS | 2,92 | 1,62 | 1,00 | 10,28 | 375,93 | 4,76 |

Erläuterung zu den jeweiligen Spalten der Tabelle:
- Spalte 1:: Quotient zur Ermittlung der RNA Reinheit
- Spalte 2:: Quotient zur Ermittlung der RNA Reinheit
- Spalte 3:: RNA Konzentration [µg/µl]
- Spalte 4:: RNA Ausbeute bestimmt aus RNA Konzentration bezogen auf eingewogenes (mg) Feuchtgewicht (FW) der Probe.
- Spalte 5:: Prozentuale Erhöhung der Ausbeute gegenüber der nach dem Stand der Technik erzielten Ausbeute an RNA
- Spalte 6:: Faktor zur Bestimmung der Erhöhung der Ausbeute; errechnet durch Division der RNA Ausbeute der jeweiligen Probe in Spalte 4 "mit HiSS" durch RNA Ausbeute in Spalte 4 "ohne HiSS"

Wie aus Tabelle 1 ersichtlich, wird durch das erfindungsgemäße Verfahren eine deutliche Steigerung der RNA Ausbeute ermöglicht. Hier können je nach Probe Steigerungen der RNA Ausbeute (ng/mg FW) von bis zu 375% erreicht werden.

**Tabelle 2:**

| RNA Isolation aus Proben von *Populus spec.* jeweils mit dem erfindungsgemäßen Verfahren (mit HiSS = Hochsalzlösung wurde zugegeben, n= 31: 31 Proben wurden untersucht) und einem nach dem Stand der Technik bekannten Verfahren (ohne HiSS = ohne Hochsalzlösung, mit dem RNeasy Mini Kit zur RNA Isolation von Qiagen, n= 5: 5 Proben wurden untersucht) | | | | | |
|---|---|---|---|---|---|
| | 1 | 2 | 3 | 4 | 5 |
| Probe | **OD₂₆₀/OD₂₃₀** | **OD₂₆₀/OD₂₈₀** | **c (ng/mg FW)** | **Ausbeuteerhöhung (%)** | **Faktor** |
| mit HiSS (n= 31) | 2,07 | 1,67 | 13,89 | | |
| ohne HiSS (n = 5) | 1,35 | 1,59 | 1,70 | 715,47 | 8,15 |

Erläuterung zu den jeweiligen Spalten der Tabelle:
- Spalte 1:: Quotient zur Ermittlung der RNA Reinheit
- Spalte 2:: Quotient zur Ermittlung der RNA Reinheit
- Spalte 3:: RNA Ausbeute bestimmt aus RNA Konzentration bezogen auf eingewogenes (mg) Feuchtgewicht (FW) der Probe
- Spalte 4:: Prozentuale Erhöhung der Ausbeute gegenüber der nach dem Stand der Technik erzielten Ausbeute an RNA
- Spalte 5:: Faktor zur Bestimmung der Erhöhung der Ausbeute; errechnet durch Division der RNA Ausbeute in Spalte 4 "mit HiSS" durch RNA Ausbeute in Spalte 4 "ohne HiSS"

Wie aus Tabelle 2 ersichtlich können auch für *Populus spec.* deutliche Ausbeuteerhöhungen von 715% an RNA erzielt werden. Auch hinsichtlich der Reinheit der RNA konnte eine Verbesserung durch das erfindungsgemäße Verfahren erreicht werden.

## Patentansprüche

1. Verfahren zur Isolierung von Nukleinsäuren aus nukleinsäurehaltigen Proben, die einen hohen Gehalt an Primär- und Sekundärmetaboliten oder Polysacchariden aufweisen, umfassend einen mechanischen Aufschluss der Proben mit bekannten Verfahren, Zugabe von einem chaotropen Denaturierungsmittel zur Probe, Homogenisieren der lysierten Probe und Entfernen der Zellreste durch Zentrifugation, Zugabe von Alkohol, Abtrennung der Nukleinsäuren durch Bindung der Nukleinsäuren an einen mineralischen Träger, Waschen sowie abschließendes Waschen und Eluieren der adsorbierten Gesamtnukleinsäure vom mineralischen Träger nach bekannten Verfahren,
**dadurch gekennzeichnet, dass**
vor dem Eluieren der adsorbierten Gesamtnukleinsäure eine Zugabe einer 0,8M NaCitrat/1,2M NaCl-Lösung erfolgt.

2. Verfahren nach Anspruch 1,
**dadurch gekennzeichnet,**
**dass** das Denaturierungsmittel Guanidinhydrochlorid, Guanidin-Isothiocyanat bzw. Guanidin-Thiocynat umfasst.

3. Verfahren nach einem der Ansprüche 1 bis 2,
**dadurch gekennzeichnet,**
**dass** das Homogenisieren der Probe durch Zentrifugation erfolgt.

4. Verfahren nach einem der Ansprüche 1 bis 3,
**dadurch gekennzeichnet,**
**dass** als Alkohol Ethanol eingesetzt wird.

5. Verfahren nach einem der Ansprüche 1 bis 4,
**dadurch gekennzeichnet,**
**dass** zur Abtrennung der Nukleinsäuren Trennsäulen auf Basis von Silikat eingesetzt werden.

6. Testkit zur Isolierung von Nukleinsäuren aus nukleinsäurehaltigen Proben, die einen hohen Gehalt an Primär- und Sekundärmetaboliten oder Polysacchariden aufweisen, umfassend Puffer, enthaltend Guanidinhydrochlorid, Guanidinisothiocyanat oder Guanidinthiocyanat, Ethanol, Trennsäule für Nukleinsäuren, Waschpuffer sowie Auffanggefäβe,
**dadurch gekennzeichnet, dass** es
eine 0,8M NaCitrat/1,2M NaCl-Lösung aufweist.

## Claims

1. Process for isolating nucleic acids from nucleic acid containing samples, which have a high content of primary and secondary metabolites or polysaccharides, comprising mechanical opening of the samples with known processes, adding a chaotropic denaturing agent to the sample, homogenising the lysed sample and removing the cell residue by centrifuging, adding alcohol, separating the nucleic acids by bonding the nucleic acids to a mineral carrier, washing and finally washing and eluting the adsorbed whole nucleic acids from the mineral carrier according to known processes,
**characterised in that**
a 0.8 M sodium citrate/1.2 M NaCl solution is added before eluting the adsorbed whole nucleic acids.

2. Process according to claim 1,
**characterised in that**
the denaturing agent comprises guanidine hydrochloride, guanidine isothiocyanate or guanidine thiocyanate.

3. Process according to claims 1 to 2,
**characterised in that**
the samples are homogenised by centrifuging.

4. Process according to claims 1 to 3,
**characterised in that**
ethanol is used as alcohol.

5. Process according to one of claims 1 to 4,
**characterised in that**
separating columns based on silicate are used to separate the nucleic acids.

6. Test kit for isolating nucleic acids from nucleic acid containing samples, which have a high content of primary and secondary metabolites or polysaccharides, including buffers, containing guanidine hydrochloride, guanidine isothiocyanate or guanidine thiocyanate, ethanol, separating columns for nucleic acids, washing buffers and receiving containers,
**characterised in that**
it has a 0.8 M sodium citrate/1.2 M NaCl solution.

## Revendications

1. Procédé d'isolement d'acides nucléiques à partir d'échantillons contenant des acides nucléiques, qui présentent une teneur élevée en métabolites primaires et secondaires ou en polysaccharides, comprenant un traitement mécanique des échantillons avec un procédé connu, l'addition d'un agent dénaturant chaotropique à l'échantillon, l'homogénéisation de l'échantillon lysé et l'élimination des résidus cellulaires par centrifugation, addition d'alcool, séparation des acides nucléiques par liaison des acides nucléiques sur un support minéral, lavage et lavage final et élution de l'acide nucléique total adsorbé du support minéral selon un procédé connu,
**caractérisé en ce que**
avant l'élution de l'acide nucléique total adsorbé, on effectue une addition d'une solution de citrate de Na 0,8 M/ NaCl 1,2 M.

2. Procédé selon la revendication 1,
**caractérisé en ce**
**que** l'agent dénaturant comprend le chlorhydrate de guanidine, l'isothiocyanate de guanidine ou le thiocyanate de guanidine.

3. Procédé selon l'une des revendications 1 à 2,
**caractérisé en ce**
**que** l'homogénéisation de l'échantillon s'effectue par centrifugation.

4. Procédé selon l'une des revendications 1 à 3,
**caractérisé en ce**
**que** l'on utilise comme alcool, de l'éthanol.

5. Procédé selon l'une des revendications 1 à 4,
**caractérisé en ce**
**que**, pour la séparation des acides nucléiques, on utilise des colonnes de séparation à base de silicate.

6. Kit de test pour isoler des acides nucléiques à partir d'échantillons contenant des acides nucléiques, qui présentent une teneur élevée en métabolites primaires et secondaires ou en polysaccharides, comprenant un tampon, contenant du chlorhydrate de guanidine, de l'isothiocyanate de guanidine ou du thiocyanate de guanidine, de l'éthanol, une colonne de séparation d'acides nucléiques, un tampon de lavage et des récipients de capture,
**caractérisé en ce**
**qu'**il présente une solution de citrate de Na 0,8 M/ NaCl 1,2 M.
